(19) Europäisches Patentamt
European Patent Office
Office européen des brevets

(11) Veröffentlichungsnummer: **0 220 387**
**B1**

(12) # EUROPÄISCHE PATENTSCHRIFT

(45) Veröffentlichungstag der Patentschrift: **19.09.90**

(21) Anmeldenummer: **86110114.5**

(22) Anmeldetag: **23.07.86**

(51) Int. Cl.$^5$: **A 61 K 39/02,** A 61 K 39/104, A 61 K 39/108, A 61 K 39/116, A 61 K 39/395

(54) **Konjugatimpfstoffe gegen Infektionen durch gramnegative Bakterien, Verfahren zu deren Herstellung sowie Verwendung derselben.**

(30) Priorität: **27.09.85 CH 4199/85**

(43) Veröffentlichungstag der Anmeldung:
**06.05.87 Patentblatt 87/19**

(45) Bekanntmachung des Hinweises auf die Patenterteilung:
**19.09.90 Patentblatt 90/38**

(84) Benannte Vertragsstaaten:
**AT BE CH DE FR GB IT LI NL SE**

(56) Entgegenhaltungen:
**EP-A-0 118 831**
**US-A-4 356 170**

**INFECTION AND IMMUNITY, Band 52, Nr. 1, April 1986, Seiten 161-165, American Society for Microbiology; S.J. CRYZ, JR. et al.: "Pseudomonas aeruginosa immunotype 5 polysaccharide-toxin A conjugate vaccine"**

(73) Patentinhaber: **Schweizerisches Serum- und Impfinstitut und Institut zur Erforschung der Infektionskrankheiten**
**Rehhagstrasse 79**
**CH-3018 Bern (CH)**

(72) Erfinder: **Cryz, Stanley J., Ph.D.**
**Stampachgasse 6**
**CH-3065 Bolligen (CH)**
Erfinder: **Fürer, Emil, Dr.sc.nat.**
**Pelikanweg 9**
**CH-3074 Muri (CH)**

(74) Vertreter: **Zutter, Hans Johann Niklaus**
**EPROVA AG Forschungsinstitut Im Laternenacker 5**
**CH-8200 Schaffhausen (CH)**

**EP 0 220 387 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

(56) Entgegenhaltungen:
INFECTION AND IMMUNITY, Band 40, Nr. 1, April 1983, Seiten 245-256, American Society for Microbiology; C. CHU et al.: "Further studies on the immunogenicity of Haemophilus influenzae type b and pneumococcal type 6a polysaccharide-protein conjugates"

JOURNAL OF BIOLOGICAL CHEMISTRY, Band 258, Nr. 14, 1981, Seiten 7305-7310, US; R.C. SEID, Jr. et al.: "Preparation and characterization of detoxified lipopolysaccharide-protein conjugates"

## Beschreibung

Infektionen durch Pseudomonas aeruginosa oder Escherichia coli kommen insbesondere vor bei hospitalisierten Patienten, Opfern von Verbrennungen und Krebspatienten, die mit Immunsuppressiva behandelt werden. Sie sind eine der Hauptursachen lebensbedrohender "nosocomial" Infektionen auf engem Raum untergebrachter (hospitalisierter) Kollektive. H. Y. Reynolds et al. Pseudomonas aeruginosa infections: persisting problems and current research to find new therapies. Annals Internal Medicine *82*:819—831 (1975). Die Häufigkeit solcher Infektionen hat in den vergangenen 30 Jahren parallel mit dem weitverbreiteten Gebrauch von Antibiotika drastisch zugenommen.

Pseudomonas aeruginosa und Escherichia coli sind auch häufig Ursache von Harnweginfektionen, Otitis, Sinusitis und Meningitis.

Antikörper gegen eine Reihe somatischer Antigene von Pseudomonas aeruginosa (siehe z.B. H. E. Gilleland et al: Use of Pseudomonas aeruginosa as a protective vaccine in mice. Infection Immunity *44:* 49—54 (1984)) und gegen extrazelluläres Protein (O. R. Pavlovskis et al. Protection against experimental Pseudomonas aeruginosa infection in mice by active immunization with exotoxin A toxoids. Infection Immunity *32*: 681—689 (1981)) erwiesen sich im Tierversuch als wirksamer Infektionsschutz.

Toxin A ist das giftigste Stoffwechselprodukt von Pseudomonas aeruginosa (P.a.). Es verhindert die eukaryontische Proteinsynthese. Mutanten von P.a. mit spezifischem Toxin A-Mangel sind weniger virulent als ihre Mutterstämme (D. E. Ohman et al. Corneal infections in mice with toxin A and elastase mutants of P.a. J. Infectious Diseases *142*: 547—555 (1980)). Passiv verabreichte oder durch aktive Vaccination erzeugte Antitoxin A-Antikörper erzeugen einen deutlich nachweisbaren Schutz gegen experimentell erzeugte P.a. Infektionen (vergl. O. R. Pavlovskis et al, loc. cit und Infection Immunity *18*: 596—602 (1977)). Weitere Untersuchungen haben einen direkten Zusammenhang zwischen Gehalt an Antitoxin-Antikörper und Ueberlebensrate von an P.a.-Bakteriämie erkrankten Patienten gezeigt (M. S. Pollack et al. Protective activity of antibodies to exotoxin A and lipopolysaccharide at the onset of P.a. septicemia in man. J. Clinical Investigation *63*: 276—286 (1979)).

Die Virulenz von P.a. bei durch Verbrennungen traumatisierten Mäusen ist abhängig vom Vorhandensein eines vollständigen "smooth-type" Lipopolysaccharid-Moleküls (S. J. Cryz et al, Infection Immunity *44*: 508—513 (1984)).

Die Schutzwirkung von serotyp-spezifischen Anti-Lipopolysaccharid-Antikörper gegen experimentelle P.a.-Infektionen ist hinlänglich dokumentiert. (Vergl. z.B. S. J. Cryz et al, Infection Immunity *43*: 795—799 (1984)).

Erhöhte Anti-Lipopolysaccharid-Antikörper-Titer erhöhen die Ueberlebenszeit von an P.a.-Sepsis erkrankten Patienten deutlich (A. S. Cross et al, J. Infectious Diseases *142*: 538—546 (1980)). Klinische Versuche mit Impfstoffen auf P.a.-Lipopolysaccharid-Basis ergaben vielversprechende Resultate (J. W. Alexander et al, J. Infectious Diseases *130* (Suppl.): S152—S158 (1974). Obwohl P.a.-Lipopolysaccharid (LPS) protektive serotypen-spezifische Antigen-determinanten enthält, ist es für den praktischen Gebrauch infolge seiner zu hohen Toxizität beim Menschen, als parenteraler Impfstoff nicht verwendbar (J. E. Pennington, J. Infectious Diseases *130* (Suppl.): S159—S162 (1974); M. D. Haghbin et al, Cancer *32*: 761—762 (1973)).

Die serotypen-spezifischen Antigen-determinanten von P.a. sind im O-Polysaccharid (PS)-Teil des LPS Moleküls untergebracht. Das Polysaccharid (PS) kann von der toxischen Lipid-A Hälfte des Lipopolysaccharides (LPS) getrennt und isoliert werden. Obwohl das PS die Antigen-determinanten enthält, kann PS nicht als Impfstoff verwendet werden, da PS nicht-immunogen ist (G. B. Pier et al, Infektion Immunity *22*: 919—925 (1978)).

Bis heute existiert kein wirksamer Impfstoff für die Verhütung von P.a.-Infektionen und Infektionen durch ähnliche gramnegative Bakterien wie z.B. Escherichia coli (E.c.).

Angesichts der relativen Häufigkeit von Infektionen durch P.a. oder E.c. und deren Gefährlichkeit ist dies ein erheblicher Mangel.

Abgesehen von der Notwendigkeit, einen wirksamen Impfstoff für die aktive Immunisierung gegen solche Infektionen bereitzustellen, besteht ein dringendes Bedürfnis nach Immunglobulinen aus spezifischen Hyperimmunseren, die einen hohen Antikörper-Titer gegen gramnegative Bakterien, insbesondere P.a. und E.c. enthalten. Derartige Seren oder Immunglobuline allein können in Fällen von Bakteriämie oder Sepsis Rettung bringen.

Antibiotika versagen oft infolge Resistenz der Keime.

Ziel der vorliegenden Erfindung ist, diese gravierenden Lücken in der medizinischen Versorgung von Infektionskranken zu schliessen. Dies ist offensichtlich nur mit einem Konjugatimpfstoff möglich.

Konjugatimpfstoffe, erhalten insbesondere durch kovalente Verknüpfung etwa von Lipopolysacchariden mit menschlichem Serumalbumin, sind z.B. aus der US—A—4,185,090 bekannt.

Methoden für die kovalente Verknüpfung von artspezifischen Polysacchariden mit artspezifischen Proteinen sind gut bekannt und verbreitet.

In der EP—A—118,831 werden immunisierende Kompositionen gegen gramnegative Bakterien beschrieben, die durch kovalente Kupplung von entgiftetem Endoprotein mit entgiftetem Polysaccharid derselben Bakterienart erhalten wurden.

In diesem Dokument werden als Proteine ausschliesslich Membran-Proteine der äusseren Membran

von gramnegativen Bakterien verwendet, während gemäss vorliegender Erfindung als Protein ausschliesslich Exotoxine, nämlich Toxin A aus einem Pseudomonas aeruginosa Stamm, ein Tetanustoxoid oder ein Diphtherietoxoid, verwendet werden.

Die Wirkung des erfindungsgemässen Impfstoffes richtet sich sowohl gegen das Bakterium (Endotoxin) wie auch gegen das Exotoxin von *Pseudomonas aeruginosa* (Toxin A) oder gegen das Tetanustoxin oder Diphtherietoxin, während die nach EP—A—118,831 erhaltenen Impfstoffe nur gegen das Bakterium gerichtet sind.

In der US—A—4,356,170 werden Kapsel-Polysaccharide (das sind Bestandteile der äusseren Membran eines Bakteriums) von Haemophiles influenza, Pneumokokken, β-hemolysierende Streptokokken und Escherichia coli benutzt, während gemäss vorliegen der Erfindung als Polysaccharid das O-Polysaccharid verwendet wird, das von den Lipopolysacchariden von Pseudomonas aeruginosa und Escherichia coli stammt, wobei die toxische Lipid-A-Hälfte abgespalten und abgetrennt wurde.

In der Publikation von Seid et al., J. Biol. Chem. 258, 7305—7310 (1981) wird durch Behandlung mit Natriumhydroxid entgiftetes Lipopolysaccharid benutzt. Gemäss den Autoren ist dies eine Folge der Deacylierung und Depolymerisierung der LPS.

Auf diese Art detoxifizierte LPS bestehen immer noch aus den Domänen I, II und III, wobei vorwiegend die toxische Domäne III deacyliert wurde.

Im Gegensatz dazu wird gemäss vorliegender Erfindung das LPS-Molekül durch Behandlung mit schwachen Säuren an der KDO-Verknüpfungsstelle gespalten und die toxische Domäne III wird entfernt. Dementsprechend werden die erfindungsgemässen Konjugate nur mit dem Restmolekül (O-Polysaccharid) bestehend aus den Domänen I+II hergestellt.

Die Wirksamkeit der vorbekannten Konjugatimpfstoffe ist bescheiden und ist klinisch nicht erprobt.

Es wurde nun gefunden, dass man ungiftige und gleichzeitig hochwirksame Konjugatimpfstoffe erhält, wenn man aus dem Endotoxin von Pseudomonas aeruginosa (P.a.) oder Escherichia coli (E.c.) isoliertes Lipid-A-freies O-Polysaccharid mit einem Protein, und zwar einem bakteriellen Endotoxin oder Exotoxoid, bestehend aus Toxin A von Pseudomonas aeruginosa, einem Tetanus- oder Diphtherietoxoid kovalent verknüpft. Die erhaltenen Konjugatimpfstoffe erwiesen sich als ungiftig und als immunogen. Sie erzeugten einen wirksamen Schutz gegen experimentell erzeugte P.a.- und E.c.-Infektionen.

Gegenstand der Erfindung ist demnach ein ungiftiger, gegen Infektionen durch Pseudomonas aeruginosa und Escherichia coli Baktieren wirksamer Konjugatimpfstoff, bestehend aus einem art- und serotypspezifischen Lipid-A-freien O-Polysaccharid aus dem Endotoxin von Pseudomonas aeruginosa oder Escherichia coli und einem Protein, welche kovalent miteinander verknüpft sind, dadurch gekennzeichnet, dass das Protein ein bakterielles Exotoxin oder Exotoxoid, bestehend aus Toxin A von Pseudomonas aeruginosa, einem Tetanus- oder Diphtherietoxoid ist.

Das Verfahren zur Herstellung dieses neuen Konjugatimpfstoffes ist dadurch gekennzeichnet, dass man ein Lipid-A-freies O-Polysaccharid aus dem Endotoxin von Pseudomonas aeruginosa oder Escherichia coli mit einem Exoprotein, bestehend aus Toxin A von Pseudomonas aeruginosa, einem Tetanus- oder Diphtherietoxoid, kovalent verknüpft.

Gegenstand der Erfindung ist auch ein Konjugatimpfstoff, der dadurch gekennzeichnet ist, dass er aus einer Mischung von 3 bis 15 Konjugaten besteht, gebildet aus 3 bis 15 serotypspezifischen Lipid-A-freien O-Polysacchariden aus dem Endotoxin von unterschiedlichen Stämmen von Pseudomonas aeruginosa oder Escherichia coli, die einzeln mit einem Exotoxin oder Exotoxoid, bestehend aus dem Toxin A von Pseudomonas aeruginosa, einem Tetanustoxoid oder Diphtherietoxoid, kovalent verknüpft sind.

Die Erfindung betrifft auch einen Konjugatimpfstoff gegen Infektionen durch Pseudomonas aeruginosa Bakterien, der dadurch gekennzeichnet ist, dass zu dessen Herstellung O-Polysaccharide aus mindestens den O-Serotypen PA 220 (Habs Serotyp 6d), E 576 (Habs Serotyp 2ab), 6511 (Habs Serotyp 4), W 18 (Habs Serotyp 10), It 2 (Habs Serotyp 11), 8505 (Habs Serotyp 3), PA 53 (Habs Serotyp 1) und It 6 (Habs Serotyp 8) von Pseudomonas aeruginosa verwendet werden.

Die Erfindung betrifft ausserdem einen Konjugatimpfstoff gegen Infektionen durch Escherichia coli Bakterien, der dadurch gekennzeichnet ist, dass zu dessen Herstellung O-Polysaccharide aus mindestens den O-Serotypen 1, 2, 4, 6, 7, 8, 11, 16, 18, 22, 25, 62 und 75 von Escherichia coli verwendet werden.

Ein weiterer Gegenstand der Erfindung ist ein polyvalenter Impfstoff, der dadurch gekennzeichnet ist, dass er aus einer Mischung besteht, die mehrere Konjugate aus serotypspezifischen O-Polysacchariden von Pseudomonas aeruginosa und von Escherichia coli mit einem Exoprotein, bestehend aus Toxin A von Pseudomonas aeruginosa, einem Tetanustoxoid oder Diphtherietoxoid, enthält und dass er Antikörper gegen die entsprechenden Serotypen von Pseudomonas aeruginosa und Escherichia coli erzeugt.

Ein weiterer Gegenstand der Erfindung ist schliesslich die Verwendung der neuen Konjugatimpfstoffe zur Herstellung von Hyperimmunseren, die ihrerseits zur Herstellung von intravenös und intramuskulär verabreichbarem Immunglobulin dienen.

Beschreibung der Impfstoffgewinnung am Beispiel eines Pseudomonas aeruginosa-Impfstoffes.

Gewinnung des Endotoxins (PS):

Für die Herstellung des erfindungsgemässen Impfstoffes gegen P.a. wird ein ganz bestimmter Serotyp von P.a. als Quelle für die Gewinnung des Endotoxins benutzt. Aus den Bakterien wird das Lipopolysaccharid (LPS) beispielsweise durch Phenol-Wasser Extraktion nach O. Westphal et al, Zeitschr.

Naturforschung: 148—155 (1952) gewonnen. Erhaltenes LPS enthält weniger als 1 Gew.% Protein und Nukleinsäuren. Das O-Polysaccharid (PS) wird aus dem LPS durch Hydrolyse mit verdünnter Essigsäure bei 100° abgelöst. Methode: I. R. Chester et al, J. General Microbiology *78*: 305—318 (1973). Der unlösliche, toxische Lipid A-Teil wird durch Zentrifugation getrennt. Der Ueberstand wird mit einer Chloroform/Methanol-Lösung extrahiert, um Reste von Lipid A zu entfernen. Die wässrige Phase, welche das PS enthält, wird im Vakuum konzentriert und anschliessend chromatographisch gereinigt, wobei Polysaccharid (PS) mit einem Molekulargewicht von 10,000 bis 75,000 gesammelt und lyophilisiert wird.

Oxidation von PS:

Das erhaltene Polysaccharid (PS) wird in Wasser durch Zusatz von Natriumperjodat bei Raumtemperatur oxidiert, wobei kupplungsfähige Aldehydgruppen entstehen. Nach Zusatz von Ethylenglykol wird das Material durch Dialyse gereinigt und danach lyophilisiert.

Gewinnung von Exoprotein (Toxin A):

Toxin A mit einem Gehalt von mehr als 95% wird aus einem Pseudomonas aeruginosa Stamm, der besonders viel Toxin A liefert, nach der Methode von S. J. Cryz et al, Infection Immunity *43*: 795—799 (1984) gewonnen.

Synthese von Toxin A-Kuppler:

Zu verdünntem Toxin A wird ein aliphatisches Dicarbonsäuredihydrazid und Carbodiimid wie beispielsweise 1-Ethyl-3(3-dimethylaminopropyl)carbodiimid gefügt. Durch diese Operation werden freie Carboxylgruppen des Toxin A mit dem Dicarbonsäuredihydrazid verknüpft. Erhaltenes Produkt kann etwa durch die folgende Formel illustriert werden:

$$\text{(Toxin A)—CO—NH—NH—CO(CH}_2)_{1-6}\text{—CO—NH—NH}_2$$

Synthese von Pseudomonas-Polysaccharid-Toxin A-Konjugat:

Der erhaltene Toxin A-Kuppler wird mit dem oxidierten, Aldehydgruppen aufweisenden, PS verknüpft, indem man äquivalente Mengen von Toxin A-Kuppler und oxidiertes PS mischt und erhaltene Schiff'sche Base (Hydrazon) mit einem Alkaliborhydrid wie z.B. mit $NaBH_4$ oder $NaBH_3CN$ reduziert.

Dabei bildet sich ein stabiles Konjugat, dessen nicht aufgeklärte Struktur man sich u.a. schematisch etwa wie folgt vorstellen kann:

$$\text{(Toxin A)—CO—NH—NH—CO—(CH}_2)_{1-6}\text{—CO—NH—NH—(Polysaccharid)}$$

Das Konjugat wird durch Dialyse gereinigt, unlösliches Material durch Zentrifugation abgetrennt und die erhaltene Mischung durch Chromatographie an einer Agarose-Säule getrennt. Material mit einem Molekulargewicht von mehr als 350,000, welches das Molekulargewicht von PS und von Toxin A deutlich übertrifft und welche das PS-Toxin A-Konjugat darstellt, wird abgetrennt und lyophilisiert.

Synthese von Polysaccharid-Tetanustoxoid-Konjugat

Tetanustoxoid

Ein für die Anwendung an Menschen bestimmtes Tetanustoxoid—z.B. TE Anatoxin(R) des Schweiz. Serum- und Impfinstitutes—wird durch Ionenaustauscher-Chromatographie an Cellulose und Gelfiltration an Agarose gereinigt, derart, dass es zu 90% aus Tetanustoxoid besteht.

Kupplung

Diese erfolgt hier beispielsweise nach der allgemeinen Methode von Jerker Porath, Methods in Enzymology Vol. *34*. S. 21 ff.

Polysaccharid (PS), welches aus LPS durch Hydrolyse gewonnen wurde, wird für diese Kupplung nicht oxidiert, sondern in wässriger Lösung in an sich bekannter Weise in alkalischem Milieu bei Raumtemperatur mit Bromcyan umgesetzt. Bromcyan (BrCN) reagiert dabei mit freien —OH Gruppen des Polysaccharides, im folgenden schematisch formuliert als (PS)—OH. Die so erhaltenen aktivierten Polysaccharide lässt man mit einem Alkylendicarbonsäuredihydrazid (z.B. $H_2NNHCO—(CH_2)_{1-6}—CONH—NH_2$) wie etwa mit Adipinsäuredihydrazid (ADH) reagieren. Dabei wird das Dihydrazid kovalent mit dem Polysaccharid verbunden. Die bei der Verknüpfung sich abspielenden Reaktionen lassen sich unter anderem z.B. etwa wie folgt illustrieren:

$$\text{(PS)—OH+BrCN}\rightarrow\text{(PS)—O—C}\equiv\text{N+H}_2\text{N—NHCO—(CH}_2)_{1-6}\text{—CO—NH—NH}_2\rightarrow$$
$$\text{(PS)—O—C(NH)—NH—NH—CO—(CH}_2)_{1-6}\text{—CO—NH—NH}_2\text{+H}_2\text{O}\rightarrow$$
$$\text{(PS)—O—CO—NH—NH—CO—(CH}_2)_{1-6}\text{—CO—NH—NH}_2$$

Das dabei erhaltene PS-Dihydrazid-Konjugat wird durch ausgiebiges Dialysieren gereinigt. Durch Zusatz einer verdünnten Mineralsäure wird die Lösung schwach angesäuert, mit einer äquivalenten Menge von Tetanustoxoid und einem Carbodiimid versetzt und einige Stunden bei Raumtemperatur gerührt. Durch die Umsetzung mit dem Toxoid (schematisch formuliert als HOCO—(Toxoid)) in Gegenwart von Carbodiimid, wobei freie Carboxylgruppen des Toxoids mit freien Hydrazid-gruppen des PS-Dihydrazid-Konjugates kondensiert werden, wird das gewünschte PS-Dihydrazid-Toxoid-Konjugat erzeugt, dessen nicht aufgeklärte Struktur man sich schematisch etwa wie folgt vorstellen kann:

$$(PS)—O—CO—NH—NH—CO—(CH_2)_{1-6}—CO—NH—NH—CO—(Toxoid)$$

Die erhaltene Mischung wird gegen einen Phosphatpuffer dialysiert und über eine Agarose-Säule chromatographiert. Material mit einem Molekulargewicht von über 350,000, welches das Molekulargewicht der Ausgangskomponenten deutlich übertrifft, wird abgetrennt und lyophilisiert.

Bei der vorstehenden Synthese kann das Tetanustoxoid auch durch Diphtherietoxoid ersetzt werden.

In analoger Weise, wie oben beschrieben, lassen sich auch Impfstoffe gegen andere gramnegative Bakterien wie zum Beispiel gegen Infektionen durch Escherichia coli erzeugen.

Nachweis von Sicherheit und Wirksamkeit der erfindungsgemässen Konjugatimpfstoffe

Eigenschaften der erfindungsgemässen Pseudomonas (PS-Toxin A)-, (PS-Tetanustoxoid)- und (PS-Diphtherietoxoid)-Konjugate:

Aus den Daten der Tabelle 1 (Seite 14) ist ersichtlich, dass die Konjugate ein Molekulargewicht von mehr als 350,000 aufweisen und damit das Molekulargewicht der Ausgangsstoffe bei weitem übertreffen. Durch die kovalente Bindung des Toxins A wird dieses hochgiftige Agens entgiftet. Die lethale Dosis steigt dabei um mindestens das 1,000fache von 0,2 µg/Maus auf mehr als 200 µg/Maus. Die Pyrogenizität ist bei den Konjugaten vergleichsweise zu der von nativem Pseudomonas aeruginosa PA 220 LPS (0,7 µg/ml/kg Körpergewicht) praktisch verschwunden.

Die Konjugate induzieren spezifische Antikörper-Antworten sowohl gegen das Polysaccharid als auch gegen den Protein-Träger, d.h. gegen Toxin A, gegen Tetanus-toxin oder gegen Diphtherie-toxin. Durch die kovalente Bindung von Toxin A mit dem Polysaccharid wird das Toxin A vollständig detoxifiziert, ohne dabei seine immunogene Aktivität zu verlieren.

Die Fähigkeit von (PS-Toxin A)-Konjugat=(PS-Toxin A)-Konjugat-impfstoff Mäuse gegen tödliche Vergiftungen mit gereinigtem Toxin A zu schützen, konnte z.B. wie folgt nachgewiesen werden:

Gruppen von Mäusen wurden bei Versuchsbeginn und am 14. Versuchstag entweder 10 µg Protein in Form entsprechender Mengen von (PS-Toxin A)-Konjugat oder ein Puffer (Kontrollgruppe) intramuskulär appliziert. Am 28. Versuchstag wurden den Mäusen graduell ansteigende Dosen von Toxin A verabreicht. Die durchschnittliche letale Dosis für die Kontrolltiere war 0,2 µg, während die mittlere lethale Dosis der mit (PS-Toxin A)-Konjugat vorbehandelten Tiere 4,7 µg/Maus betrug.

Wirksamkeit von (PS-Tetanustoxoid)- und von (PS-Toxin A)-Konjugat als Impfstoff.

Die Fähigkeit von nativem Lipopolysaccharid (LPS aus Pseudomonas aeruginosa), (PS-Tetanustoxoid)-Konjugat und von (PS-Toxin A)-Konjugat Versuchstiere gegen experimentell verursachte Pseudomonas aeruginosa-Brandwunden-Sepsis zu schützen, wird anhand der Tabelle 2, Seite 15 demonstriert.

TABELLE 1:
Eigenschaften von Pseudomonas (PS-Toxin A)- und (PS-Tetanustoxoid)-Konjugat

| | PS | Tetanustoxoid | Toxin A | PS-Toxin A Konjugat | PS-Tetanustoxoid Konjugat |
|---|---|---|---|---|---|
| Molekulargewicht[1)] | <70,000 | 150,000 | 66,000 | >350,000 | >350,000 |
| Toxizität[2)] | ungiftig | ungiftig | 0.2 µg | ungiftig | ungiftig |
| Pyrogenizität[3)] | 50 µg | 50 µg | — | 50 µg | 85 µg |
| Immunogenität[4)] | nicht-immunogen | Immunogen (Anti-Tetanus IgG) | Immunogen (Anti-Toxin A IgG) | Immunogen (Anti-PS und Anti-Toxin A IgG) | Immunogen (Anti-PS und Anti-Tetanustoxin IgG) |

[1)] bestimmt durch Gelfiltration über eine AcA34-Kolonne.

[2)] Bestimmt durch intraperitoneale Injektion in 18—20 g schwere weibliche Mäuse, ausgedrückt als die durchschnittliche tödliche Dosis pro Maus. Ungiftig bedeutet, dass ein Minimum von 50 µg intraperitoneal verabreichtes Antigen keine Todesfälle verursacht.

[3)] Die höchste an Kaninchen i.v. verabreichte Antigendosis, welche ein Anstieg der Körpertemperatur von höchsten 0.3°C verursacht, ausgedrückt als µg/ml/kg-Körpergewicht.

[4)] Bestimmt durch Immunisierung von Gruppen aus jeweils 3 Kaninchen mit 50 µg Antigen pro Kaninchen. Die Seren der Tiere wurden auf Vorhandensein spezifischer IgG (Immunoglobulin)-Antikörper mittels der *E*nzyme-*l*inked-*i*mmunosorbent-*a*ssay-[ELISA]-Technik analysiert.

TABELLE 2:

Schutz gegen tödliche durch Pseudomonas aeruginosa PA 220-induzierte Brandwundensepsis durch Immunisierung mit LPS von PA 220, (PS-Tetanustoxoid)-Konjugat und von (PS-Toxin A)-Konjugat.

| Immunogen[1] | $LD_{50}$[2] |
|---|---|
| Keines (Kontrollen) | 20 |
| LPS | $10^6$ |
| PS-Tetanustoxoid-Konjugat | $10^6$ |
| PS-Toxin A-Konjugat | $10^6$ |

[1] Mäuse erhielten 1 µg i.m. vor Versuchsbeginn und am 14. Versuchstag. Sie wunden anschliessend einem Verbrennungstrauma unterworfen (Methode: I.A. Holder et al, Infection Immunity *35.* 276—280 (1982) und am 28. Tag mit steigenden Dosen von P. aeruginosa PA 220 belastet.

[2] $LD_{50}$=mittlere lethale Dosis von P. aeruginosa PA 220, berechnet nach der Methode von L. J. Reed und H. A. Muench, American J. Hygiene *27.* 493—497 (1938).

Die Daten wiesen nach, dass die Immunisierung mit LPS, (PS-Tetanustoxoid)-Konjugat und mit (PS-Toxin A)-Konjugat eine etwa gleich starke Schutzwirkung gegen fatale P.a. Sepsis erzeugt. Die lethale Dosis für P. aeruginosa PA 220 wird dadurch gegenüber der nicht immunisierten Kontrollgruppe um das 50 000fache erhöht.

Sicherheit und Immunogenität von (PS-Tetanustoxoid)-Konjugatimpfstoff bei Menschen.

Gesunde 16- bis 59-jährige erwachsene Freiwillige beiderlei Geschlechtes erhalten bei Versuchsbeginn und nach 14 Tagen 100 µg des Konjugatimpfstoffes in 0.5 ml subkutan in den Oberarm appliziert.

Alle Reaktionen der Versuchspersonen wurden registriert. Nebenreaktionen waren selten, schwach und flüchtig.

Venöse Blutproben wurden bei Versuchsbeginn und 14 und 28 Tage nach der Impfung gezogen. Die aus den Blutproben gewonnenen Seren wurden gesammelt und bei −20°C aufbewahrt. Die Immunglobulin G (IgG)-Antikörpertiter wurden bestimmt.

Die Immunisierung mit (PS-Tetanustoxoid)-Konjugat führte zu einem signifikanten Anstieg des mittleren Anti-PA220 LPS IgG-Titers nach 14 und 28 Tagen seit der Impfung verglichen mit dem Ausgangstiter. Siehe Tabelle 3, Seite 18.

Ueber 80% der Versuchspersonen reagierten mit einer um das 4fache oder mehr erhöhten Antikörpertiter. Die Immunisierung mit diesem Konjugatimpfstoff führte auch zu einem Tetanustoxin neutralisierenden Anti-Tetanus-Antikörper-Titer.

Serum-Pools wurden erhalten durch Kombination einer äquivalenten Menge Serum von jeder Versuchsperson bei Versuchsbeginn (Prä-Immun-Pool) und nach 28 Tagen (Immun-Pool).

Der Prä-Immun-Pool enthielt 2,7 internationale Tetanustoxin neutralisierende Einheiten pro ml, während der Immun-Pool 6,2 Einheiten pro ml aufwies.

Anti-PA220-LPS-IgG-Antikörper, welche durch Impfung von Menschen mit dem erfindungsgemässen (PS-Tetanustoxoid)-Konjugat erzeug wurden, erwiesen sich als hochwirksam gegen fatale experimentelle durch P. aeruginosa verursachte Sepsis. Passiv transferierter Post-Immun-IgG war für die Verhütung von Todesfällen signifikant wirksamer als passiv transferierter Prä-Immun-IgG. Der gemessene Schutz korreliert mit dem Anti-PA220-LPS-IgG-Titer der IgG-Präparate. Tabelle 4 siehe Seite 19.

(PS-Tetanustoxoid)-Konjugat ist demnach ein Impfstoff, der sich beim Menschen gefahrlos anwenden lässt und bei mehr als 80% der Geimpften einen signifikant erhöhten Anti-PA220-LPS-IgG-Titer erzeugt. Das auf diese Weise im Menschen erzeugte Anti-PA220-LPS-Immunglobulin erwies sich als hochwirksam gegen fatale P.-aeruginosa-Sepsis.

Analoge Ergebnisse werden auch mit den erfindungsgemässen

P.a.(PS)-Toxin A-,
P.a.(PS)-Diphtherietoxoid-,
E.c.(PS)-Toxin A-,
E.c.(PS)-Tetanustoxoid- und
E.c.(PS)-Diphtherietoxoid-Konjugatimpfstoffen erzielt.

TABELLE 3:

Anti-PA220 LPS IgG-Antikörper-Antwort nach Immunisierung mit (PS-Tetanustoxoid)-Konjugat

| | Mittlerer ELISA Titer Bereich | | |
|---|---|---|---|
| | Post-Immun[2] | | ≥4fach erhöhter Titer[3] |
| Prä-Immun[1] | 14 Tage | 28 Tage | (%) |
| 43 (14—28) | 652 (39—3,200)[4] | 823 (47—4,400)[4] | 13/16 (82) |

[1] Prä-Immun=zur Zeit der Immunisierung.
[2] Post-Immun=14 bzw. 28 Tage nach der Immunisierung.
[3] Quotient aus Post- und Prä-Immun-Titer
[4] Der mittlere Post-Immun-Titer war signifikant erhöht ($p<0.01$).

TABELLE 4:

Fähigkeit von passiv transferiertem IgG Mäuse gegen fatale P. aeruginosa PA 220-Brandwunden-Sepsis zu schützen.

| Uebertragene IgG[1] | Anti-LPS IgG ELISA Titer | $LD_{50}$ |
|---|---|---|
| Keine[2] | — | $1.3\times10^1$ |
| Prä-Immun[3] | 73 | $1.3\times10^3$ |
| Post-Immun[4] | 1065 | $6.7\times10^5$ |

[1] Jede Maus erhielt i.v. etwa 160 µg humanes IgG in 0.2 ml 24 Stunden vor der Belastung mit P. aeruginosa PA 220.
[2] Die Kontrollen erhielten 0,2 ml sterile Salzlösung.
[3] Hergestellt aus aliquoten Teilen der Seren von allen freiwilligen Versuchspersonen vor der Immunisierung.
[4] Hergestellt aus aliquoten Teilen der Seren von allen freiwilligen 28 Tage nach der Immunisierung.

(PS-Tetanustoxoid)-, (PS-Diphtherietoxoid)- und (PS-Toxin A)-Konjugatimpfstoffe.

Diese Konjugate erwiesen sich in zahlreichen Tests als ungiftige, nicht pyrogene und hochwirksame Impfstoffe. Derartige Impfstoffe sind bisher nicht bekannt. Für die praktische Verwendung ist es wünschenswert, einen Impfstoff anbieten zu können, der eine breit gefächerte Wirkung gegen Infektionen durch verschiedene Serotypen etwa von Pseudomonas aeruginosa entfaltet.

Es wurde gefunden, dass diese breit gefächerte Wirkung erreicht werden kann, wenn man eine Komposition aus (PS-Tetanustoxoid)-, (PS-Diphtherietoxoid)- oder (PS-Toxin A)-Konjugaten verwendet, deren PS (Polysaccharid)-Teil aus mehreren, bei Infektionen hauptsächlich auftretenden Serotypen von Pseudomonas aeruginosa (P.a.) stammt. Dazu sind etwa 3 bis 15 verschiedene Serotypen von Pseudomonas aeruginosa notwendig.

Die Verknüpfung der Polysaccharide (PS) der verschiedenen Serotypen zum PS-Protein-Konjugat muss, um brauchbare Präparate zu erhalten, einzeln erfolgen. Das bedeutet, dass man aus 3 bis 15 reinen Stämmen von P.a. jeweils die individuelle LPS isoliert und daraus die PS und die einzelnen PS individuell mit dem Protein Teil (Toxin A, Tetanustoxoid oder Diphtherietoxoid) kovalent verknüpft, die erhaltenen Konjugate auf ihre Verträglichkeit und Wirksamkeit prüft und schliesslich die 3 bis 15 Einzelkonjugate zusammenmischt zum fertigen Kombinationspräparat.

Dieselbe Prozedur wird auch für die Herstellung eines Kombinationsimpfstoffes gegen Infektionen durch andere gramnegative Bakterien wie etwa Escherichia coli angewandt.

Impfstoffe, die gegen Infektionen durch verschiedene gramnegative Bakterienarten wirksam sind.

Es wäre sehr wünschenswert, durch eine einzige Impfung Antikörper gegen verschiedene gramnegative Bakterienarten erzeugen zu können. Besonders wichtig wäre dies dann, wenn die Antikörper geerntet und zu spezifischen gegen Infektion durch gramnegative Bakterien wirksamen Immunglobulinen verarbeitet werden sollen.

Es wurde gefunden, dass man dieses Ziel erreichen kann, wenn man den gegen eine Bakterienart

spezifischen Konjugatimpfstoff mit einem oder mehreren gegen andere Bakterienarten spezifischen Konjugatimpfstoffen mischt.

Verwendung von Konjugatimpfstoffen gemäss vorliegender Erfindung zur Herstellung von spezifischen Immunglobulinen.

Es wurde weiter oben nachgewiesen, dass die erfindungsgemässen Konjugatimpfstoffe beim Menschen die Bildung spezifischer Antikörper gegen die betreffende Bakterienart bewirken. Der auf diese Weise erzeugte Antikörpertiter im Serum der freiwilligen Impflinge ist so hoch, dass diese Seren als Ausgangsmaterial zur Herstellung von spezifischen Immunglobulinen dienen können. Damit hat der Arzt ein Mittel in der Hand, mit dem er an entsprechender Bakteriämie erkrankte, nicht geimpfte Patienten mit Erfolg behandeln und retten kann.

Beispiele

Beispiel 1

Pseudomonas aeruginosa Impfstoff: PS-Toxin A-Konjugat

A. Isolierung der LPS (IT-5=Habs 10)

500 ml 3% Trypticase-Soy-Broth (TSB) (Becton, Dickinson and Company, USA) enthaltend 1% Glycerin, werden mit einer lyophilisierten Kultur von Pseudomonas aeruginosa (IT-5=Habs 10) beimpft und bei 37°C und 150 RPM inkubiert. Mit dieser Vorkultur werden 50 lt 3% TSB Medium, enthaltend 1% Glycerin beimpft und im Fermenter 16 Stunden bei 37°C inkubiert. Die Zellen werden mit einer Durchlaufzentrifuge (z.B. von Westfalia) geerntet und zweimal mit Tris-NaCl-Puffer pH 9 gewaschen. Die Zellen werden dann in Tris-NaCl-Puffer pH 9 suspendiert und mechanisch aufgeschlossen z.B. mittels Glasbeads (Dyno-Mill, W. A. Bachhofer AG, Basel, Schweiz). Die Zellwände werden durch Zentrifugation von Cytoplasma, das verworfen wird, abgetrennt. Die Zellwände werden nochmals in Tris-NaCl-Puffer aufgeschwemmt und mittels einer Kühlzentrifuge bei 23,500 g für 30 Min. sedimentiert. Die Zellwände werden dann in 1,000 ml Wasser suspendiert und mit 1,280 ml 80% Phenol versetzt. Methode: O. Westphal, Z. Naturforsch. *7*: 148—155. Die Mischung wird auf 68—70°C erwärmt und 5 Min. bei dieser Temperatur gerührt. Darauf wird das Extraktionsgemisch im Eisbad auf 4—10°C abgekühlt und anschliessend zur Trennung der Phasen für 15 Min. bei 23,500 g in einer Kühlzentrifuge zentrifugiert. Die obere Phase entspricht der Wasserphase und enthält die Lipopolysaccharide (LPS). Sie wird sorgfältig von der Phenolphase abgetrennt, welche nochmals mit 1 Liter destilliertem Wasser versetzt wird, und das Ganze nochmals bei 68—70°C für 5 Min. gerührt. Es wird wiederum im Eisbad auf 4—10°C abgekühlt und die Phasen werden wie oben getrennt. Die wässrigen Phasen werden vereinigt und gegen Wasser solange dialysiert, bis sie frei von Phenol sind. Die Phenolphasen werden verworfen. Die Wasserphasen werden dann in der Ultrazentrifuge zum Sedimentieren der LPS bei 100,000 g während drei Stunden zentrifugiert. Das Sediment wird in 0,05 M Tris-Puffer, pH 7,2, enthaltend 0,05 M NaCl gelöst und mit RNAse, DNAse und Pronase (Boehringer, Mannheim, Westdeutschland) wie folgt behandelt. Man gibt RNAse zu einer Endkonzentration von 20 µg/ml zu und inkubiert für drei Stunden bei 37°C. Dann fügt man DNAse zu einer Endkonzentration von 20 µg/ml und $MgSO_4$ zu einer Endkonzentration von 0,1 M und inkubiert ebenfalls für drei Stunden bei 37°C. Anschliessend wird Pronase zu einer Konzentration von 200 µg/ml zugefügt, für 2—3 Stunden bei 37°C inkubiert und anschliessend bei 4°C über Nacht gegen Wasser dialysiert. Die LPS werden durch zweimalige Ultrazentrifugation weiter gereinigt und anschliessend lyophilisiert. Die so gereinigten LPS enthalten weniger als 1% Nukleinsäuren und Protein als Verunreinigung.

B. Isolierung des serotyp-spezifischen Polysaccharides

Die gereinigten LPS werden in einer Konzentration von 3 mg/ml 1 %iger Essigsäure suspendiert und für 90 Min. bei 100°C hydrolisiert. Methode: G. Schmidt et al, Eur. J. Biochem. *10*: 501—510 (1969). Mit dieser Behandlung wird der Polysaccharidteil vom toxischen Lipidteil (Lipid A) getrennt. Lipid A ist unlöslich und wird durch Zentrifugation vom löslichen Polysaccharid entfernt. Die wässrige Polysaccharidlösung wird zur Entfernung von Spuren von Lipid A dreimal mit dem gleichen Volumen Chloroform-Methanol (3:1) extrahiert und am Wasserstrahlvakuum eingeengt. Die konzentrierte PS-Lösung wird an einem Agarose-Gel z.B. ACA34-Gel (LKB-Produkter AB, Bromma, Schweden) chromatographiert. (Säule 5 cm×40 cm, Laufgeschwindigkeit 1,7 ml/min, Fraktionsgrösse 17 ml.) Polysaccharid wie in den einzelnen Fraktionen bestimmt. Die Fraktionen, die die PS mit einem Molekulargewicht von 10,000 bis 75,000 enthalten, werden gepoolt und lyophilisiert.

C. Oxydation der PS

Die lyophilisierten PS werden in destilliertem Wasser in einer Konzentration von 5 mg/ml gelöst. Dazu fügt man $NaIO_4$ (Natriumperjodat) zu einer Konzentration von 0,1 M. Man lässt die Lösung für zwei Stunden bei Raumtemperatur im Dunkeln stehen. Ueberschüssiges Oxydationsmittel wird durch Zufügen von Ethylenglycol zu einer Konzentration von 0,2 M inaktiviert. Die oxydierten PS werden erschöpfend gegen Wasser dialysiert und lyophilisiert.

D. Toxin A

Toxin A wird aus dem Ueberstand von *P. aeruginosa* Stamm PA 103 (erhalten von Dr. B. Wretlind, Karolinska Institute, Stockholm, Schweden) nach folgender bekannter Methode isoliert: Ultrafiltration,

DEAE-Cellulose-Chromatographie, Hydroxylapatit-Chromatographie (beschrieben von Cryz *et al.* in Infect. Immun. *39*: 1072—1079, 1983). Die Reinheit des so isolierten Toxin A beträgt mehr als 95%.

E. Herstellen von Toxin A-ADH (ADH=Adipinsäuredihydrazid)

ADH wird als "Spacer"-Molekül und zur Einführung von reaktionsfähigen Aminogruppen kovalent mit Toxin A wie folgt verknüpft: Reines Toxin A wird in 0,05 N Na-Phosphat-Puffer, pH 7,2 auf 5 mg/ml eingestellt. Dazu gibt man ADH (Adipinsäuredihydrazid) und 1-Ethyl-3-(3-dimethylaminopropyl)carbodiimid in fester Form zu einer Konzentration von je 10 mg/ml. Der pH wird mit verdünnter Salzsäure auf 4,8 eingestellt. Die Lösung wird vorsichtig gerührt für zwei Stunden bei 22°C und der pH durch Zugabe von weiterer 1 N Salzsäure konstant bei 4,8 gehalten. Nach beendeter Reaktion wird die Mischung erschöpfend gegen 0,05 M Na-Phosphat-Puffer pH 8,0 bei 4°C dialysiert. Unlösliches Material wird durch Zentrifugation entfernt. Die Toxin A-ADH-Lösung wird auf eine Konzentration von 5 mg/ml eingestellt.

F. Herstellen von Polysaccharid-Toxin A-Konjugat

Die Toxin A-ADH-Lösung wird für zwei Stunden bei Raumtemperatur gegen 0,5 M Na-Phosphat-Puffer dialysiert und die Toxin A-ADH-Lösung auf 5 mg/ml eingestellt. Zu dieser Lösung gibt man ein gleiches Volumen einer Lösung von 5 mg/ml oxydiertes PS ebenfalls in 0,5 M Na-Phosphat-Puffer pH 8,0 gelöst. Man lässt die Lösung für sechs Stunden bei Raumtemperatur stehen und fügt dann $NaBH_3CN$ aus einer zehnfach konzentrierten (0,5 M) Lösung bis zu einer Konzentration von 0,05 M zu. Die Mischung lässt man dann für 5—7 Tage bei Raumtemperatur stehen. Anschliessend wird erschöpfend dialysiert gegen Phosphatpuffer (PBS) von pH 7,4, enthaltend 0,02% Merthiolat.

Unlösliches Material wird durch Zentrifugation entfernt und die gelöste Reaktionsmischung über ein Agarose-Gel z.B. AcA34-Gel chromatographiert. Von den Fraktionen wird die Absorption bei 280 und 220 nm gemessen. Material, das mit dem Leervolumen ($v_o$) eluiert wird und demzufolge ein Molekulargewicht von über 350,000 besitzt, was weit über den Molgewichten der Reaktionspartner liegt, wird gesammelt und sterilfiltriert. Das Material wird so verdünnt, dass 0,5 ml einer Humandosis entspricht und dass die Endlösung 5% Lactose in 50 %iger PBS und 0,01% Merthiolat enthält, und dann lyophilisiert.

Am Impfstoff werden die folgenden Prüfungen durchgeführt:

| Prüfung | Methode |
|---|---|
| Polysaccharidgehalt | Phenol-Schwefelsäure[1] unter Verwendung des reinen Ausgangs-PS als Standard. |
| Proteingehalt | Lowry *et al.*[2] unter Verwendung von Rinderserumalbumin als Standard. |
| Sterilität | |
| Pyrogenität | Kaninchen |
| Verträglichkeit | Zwei Humandosen intraperitoneal an je zwei Meerschweinschen und eine Humandosis an je fünf Mäusen. |
| Toxizität | 200 µg (Protein) in 0,5 ml intraperitoneal in sechs Mäusen. |

Resultate siehe Tabelle 1.

[1]M. Dubois et al. Colorimetric method for determination of sugars and related substances. Anal. Chem. *28:* 350—356.

[2]O. H. Lowry et al. Protein measurement with the Folin-phenol reagent. J. Biol. Chem. *193*: 265—275 (1951).

Beispiel 2

Pseudomonas aeruginosa Impfstoff: PS-Tetanustoxoid-Konjugat

100 mg PS, hergestellt nach Beispiel 1 A/B, werden in 9,2 ml destilliertem Wasser gelöst. Dazu gibt man vorsichtig 0,44 ml einer Lösung von Bromcyan. Der pH wird mit 1 N NaOH für 6 Min. auf pH 10,5 gehalten. Danach wird der pH durch Zufügen von festem $NaHCO_3$ auf 8,6 gesenkt. 0,4 g Adipinsäuredihydrazid (ADH) wird zugegeben und die Lösung unter vorsichtigem Rühren 16 Stunden bei 4°C belassen. Nach erschöpfender Dialyse gegen Wasser werden 100 mg z.B. durch Ionenaustauscher und Gelchromatographie gereinigtes, konzentriertes oder lyophilisiertes Tetanustoxoid und 3,3 ml 1 - Ethyl - 3( - 3 - dimethylaminopropyl) - carbodiimid - Lösung (20 mg/ml) zugegeben. Der pH wird mit 0,1 N Salzsäure

auf 4,8 eingestellt und während 4 Stunden bei Raumtemperatur unter leichtem Rühren konstant gehalten. Die Reaktionsmischung wird dann erschöpfend gegen PBS-Merthiolat 0,02% dialysiert. Danach wird das Gemisch an einem Agarose-Gel (Säule 5 cm×40 cm, Laufgeschwindigkeit 1,1 ml pro Minute) mit PBS-Merthiolat-Puffer chromatographiert. Es werden Fraktionen von 11 ml gesammelt, und der Protein- und PS-Gehalt bestimmt. Material, das mit dem Leervolumen eluiert wurde und demzufolge ein Molekulargewicht von über 350,000 besitzt, wird gepoolt und wie in Beispiel 1 lyophilisiert. PS-Tetanus-Konjugat wird analog dem PS-Toxin A Konjugat geprüft (siehe Beispiel 1).

Beispiel 3

Pseudomonas aeruginosa Impfstoff: PS-Diphtherietoxoid-Konjugat

Wie im Beispiel 2 beschrieben, wird das Polysaccharid, das die serotypspezifischen Determinanten enthält, mit einem gereinigten und konzentrierten oder lyophilisierten Diphtherietoxoid kovalent verbunden.

Der erhaltene Impfstoff ist ungiftig und erzeugt serotypspezifische Antikörper gegen Pseudomonas aeruginosa sowie antitoxische Antikörper gegen Diphtherietoxoid.

Beispiel 4

Escherichia coli—PS-Toxin A-Konjugatimpfstoff

Analog wie im Beispiel 1 beschrieben, wird das Polysaccharid, das die serotypspezifischen Determinanten enthält, aus dem Endotoxin von *E. coli*-Fermenterkulturen isoliert. Wie im Beispiel 1 beschrieben, wird das Polysaccharid mit Na-Perjodat oxydiert und mit Toxin A mit Hilfe des "Spacer"-Moleküls Adipinsäuredihydrazid kovalent verbunden. Der Konjugatimpfstoff wird lyophilisiert und geprüft. Er ist ungiftig, gut verträglich und induziert die Bildung von serotypspezifischen Antikörpern gegen E. coli.

Beispiel 5

Escherichia coli—PS-Tetanustoxoid-Konjugatimpfstoff

*E. coli*-Polysaccharid, enthaltend die serotypspezifischen Determinanten, wird mit Bromcyan aktiviert und analog Beispiel 2 kovalent mit dem Spacermolekül Adipinsäuredihydrazid (ADH) verbunden. Das Polysaccharid-ADH wird mittels eines wasserlöslichen Carbodiimides kovalent mit den Carboxylgruppen des Tetanustoxoides verbunden. Erhaltener Impfstoff ist ungiftig, gut verträglich und induziert die Bildung von serotypspezifischen Antikörpern gegen E. coli sowie von antitoxischen Antikörper gegen Tetanustoxoid.

Beispiele 6—8

Mehrwertige Pseudomonas aeruginosa-Konjugatimpfstoffe

6. 8-wertiger P. aeruginosa-P.a. Toxin A-Impfstoff

Je 50—100 µg von neun serotypspezifischen Einzelkonjugaten pro Dosis werden durch Mischen zum polyvalenten Pseudomonas aeruginosa-Konjugatimpfstoff vereinigt.

Vorgehen: Die Polysaccharid-Komponenten der Konjugate werden zunächst einzeln aus dem Endotoxin der folgenden 8 Pseudomonas aeruginosa-Stämme isoliert:

PA 220 (Habs Serotyp 6d); 8505 (Habs Serotyp 3);
6511 (Habs Serotyp 4); IT-2 (Habs Serotyp 11);
E 576 (Habs Serotyp 2ab); PA 53 (Habs Serotyp 1);
W 18 (Habs Serotyp 10);
IT 6 (Habs Serotyp 8).

Die 9 verschiedenen PS werden danach einzeln mit dem Proteinteil von Exotoxin A von Pseudomonas aeruginosa gemäss Beispiel 1 kovalent verknüpft und die 8 Konjugate schliesslich zum 8-wertigen Impfstoff vermischt.

Der erhaltene 8-wertige Impfstoff hat folgende Eigenschaften:

1) Er ist ungiftig für Tiere und nicht pyrogen für Kaninchen.
2) Sein Molekulargewicht ist grösser als 350,000.
3) Der Impfstoff schütz gegen ein breites Spektrum von P. aeruginosa-Infektionen.

7. 8-wertiger P. aeruginosa-Tetanustoxoid-Impfstoff

Die Herstellung erfolgt analog Beispiel 6.

Die 8 verschiedenen PS werden einzeln mit humanem Tetanustoxoid gemäss Beispiel 2 kovalent verknüpft und danach zum mehrwertigen Impfstoff gemischt.

Der erhaltene Impfstoff induziert die Bildung von serotypspezifischen Antikörpern gegen P. aeruginosa sowie von antitoxischen Antikörpern gegen Tetanustoxin.

8. 8-wertiger P. aeruginosa-Diphtherietoxoid-Impfstoff

Die 8 im Beispiel 6 spezifizierten PS werden einzeln mit Diphtherietoxoid gemäss Beispiel 3 kovalent verknüpft und danach zum mehrwertigen Impfstoff gemischt.

Der erhaltene Impfstoff induziert die Bildung von serotypspezifischen Antikörpern gegen P. aeruginosa sowie von antitoxischen Antikörpern gegen Diphtherietoxin.

Beispiele 9—13

Mehrwertige Escherichia coli-Konjugatimpfstoffe

9. 13-wertiger E. coli-P.a. Toxin-Impfstoff

Je 50—100 µg von 13 Einzelkonjugaten, deren Polysaccharidteil vom Endotoxin der Serotypen 1, 2, 4, 6, 7, 8, 11, 16, 18, 22, 25, 62, 75 von E. coli stammt und deren Proteinteil vom Exotoxin A von Pseudomonas aeruginosa (P.a.) stammt, werden vermischt.

Erhaltener Impfstoff induziert die Bildung von serotypspezifischen Antikörpern gegen Escherichia coli sowie von antitoxischen Antikörpern gegen P.a.-Toxin.

10. 13-wertiger E. coli-Tetanustoxoid-Impfstoff

13 Einzelkonjugate aus den im Beispiel 9 genannten PS von E. coli und humanem Tetanustoxoid werden analog Beispielen 1—3 hergestellt und analog Beispielen 6/7 vermischt. Der erhaltene Impfstoff induziert die Bildung von spezifischen Antikörpern gegen E. coli und antitoxischen Antikörpern gegen Tetanustoxin.

11. 13-wertiger E. coli-Diphtherietoxoid-Konjugatimpfstoff

13 Einzelkonjugate aus den im Beispiel 9 genannten PS von E. coil und Diphtherietoxoid werden analog Beispielen 1—3 hergestellt und analog Beispielen 6/7 vermischt.

Der erhaltene Impfstoff induziert die Bildung von spezifischen Antikörpern gegen E. coli und antitoxischen Antikörpern gegen Diphtherietoxin.

12. 10-wertiger E. coli-Konjugatimpfstoff

Je 50—100 µg pro Dosis von 10 Einzelkonjugaten, deren Polysaccharidteil vom Endotoxin der O-Serotypen 1, 2, 4, 6, 7, 8, 18, 22, 25, 75 stammt und deren Proteinteil vom Exotoxin A von *P. aeruginosa* oder von einem Human-Tetanustoxoid stammt, werden vermischt.

13. 3-wertiger E. coli-Konjugatimpfstoff

Je 50—100 µg pro Dosis von 3 Einzelkonjugaten, deren Polysaccharidteil vom Endotoxin der O-Serotypen a) 1, b) 2 und c) 4 und deren Proteinteil a) vom Exotoxin A vom P. aeruginosa, b) von humanem Tetanustoxoid bzw. c) von Diphtherietoxoid stammt, werden vermischt.

Beispiel 14

Mehrwertiger Konjugatimpfstoff gegen P. aeruginosa- und E. coli-Infektionen

Dieser Impfstoff wird erhalten durch Mischen von 8 Einzelkonjugaten (50—100 µg), deren Polysaccharidteil vom *Pseudomonas aeruginosa* Endotoxin stammt, sowie von 13 Einzelkonjugaten, deren Polysaccharidteil vom Endotoxin von *E. coli* stammt. Der Proteinteil der Einzelkonjugate stammt jeweils entweder vom Exotoxin A von *P. aeruginosa* oder von einem Human-Tetanustoxoid. Erhaltener Impfstoff ist in der Lage, serotypspezifische Antikörper gegen *P. aeruginosa* sowie gegen *E. coli* zu induzieren. Im weitern induziert dieser Impfstoff auch antitoxische Antikörper, die gegen das Exotoxin A sowie gegen Tetanustoxin gerichtet sind.

Beispiel 15

Verwendung des mehrwertigen Konjugatimpfstoffes zur Herstellung von spezifischem Immunglobulin

Freiwillige werden mit dem mehrwertigen Impfstoff gemäss Beispiel 14 in den Deltoidesmuskel immunisiert. Vier Wochen danach erfolgt eine Boosterimpfung mit dem gleichen Impfstoff und Dosierung in den Deltoidesmuskel. Eine Woche später wird den Freiwilligen eine Blutprobe aus der Armvene entzogen und die Antikörpertiter gegen die im Impfstoff enthaltenen Serotypen (von *P. aeruginosa* und *E. coli*) sowie gegen Exotoxin A und Tetanustoxin bestimmt. Ist die Titerzunahme gegen alle erwähnten Antigene als Folge der Impfungen 4-mal oder mehr, so wird von den Impflingen etwa 240 ml Blut gespendet. Die Seren werden gepoolt und ein γ-Globulin-Präparat für intravenöse Applikation (IVIG) via die folgenden bekannten Schritte, beispielsweise gemäss dem Verfahren der EP—A—85,747 hergestellt: Fraktionierte Alkohol-Fällung nach Cohn, Ionenaustausch-Chromatographie, Ultrafiltration und Diafiltration. Der Proteingehalt wird auf 5% eingestellt und in einem stabilisierenden Medium lyophilisiert.

## Patentansprüche

1. Ungiftiger Konjugatimpfstoff wirksam gegen Infektionen durch Pseudomonas aeruginosa und Escherichia coli Bakterien bestehend aus einem art- und serotypspezifischen Lipid-A-freien O-Polysaccharid aus dem Endotoxin von Pseudomonas aeruginosa oder Escherichia coli und einem Protein, welche kovalent miteinander verknüpft sind, dadurch gekennzeichnet, dass das Protein ein bakterielles Exotoxin oder Exotoxoid bestehend aus Toxin A von Pseudomonas aeruginosa, einem Tetanus- oder Diphtherietoxoid ist.

2. Verfahren zur Herstellung eines Konjugatimpfstoffes nach Anspruch 1, dadurch gekennzeichnet, dass man ein Lipid-A-freies O-Polysaccharid aus dem Endotoxin von Pseudomonas aeruginosa oder Escherichia coli mit einem Exoprotein, bestehend aus Toxin A von Pseudomonas aeruginosa, einem

Tetanus- oder Diphtherietoxoid, kovalent verknüpft.

3. Konjugatimpfstoff nach Anspruch 1, dadurch gekennzeichnet, dass er aus einer Mischung von 3 bis 15 Konjugaten besteht, gebildet aus 3 bis 15 serotypspezifischen Lipid-A-freien O-Polysacchariden aus dem Endotoxin von unterschiedlichen Stämmen von Pseudomonas aeruginosa oder Escherichia coli, die einzeln mit einem Exotoxin oder Exotoxoid, bestehend aus dem Toxin A von Pseudomonas aeruginosa, einem Tetanustoxoid oder Diphtherietoxoid, kovalent verknüpft sind.

4. Konjugatimpfstoff gemäss Anspruch 3, dadurch gekennzeichnet, dass zu dessen herstellung O-Polysaccharide aus mindestens den O-Serotypen PA 220 (Habs Serotyp 6d), E 576 (Habs Serotyp 2ab), 6511 (Habs Serotyp 4), W 18 (Habs Serotyp 10), It 2 (Habs Serotyp 11), 8505 (Habs Serotyp 3), PA 53 (Habs Serotyp 1) und It 6 (Habs Serotyp 8) von Pseudomonas aeruginosa verwendet werden.

5. Konjugatimpfstoff gemäss Anspruch 3, dadurch gekennzeichnet, dass zu dessen Herstellung O-Polysaccharide aus mindestens den O-Serotypen 1, 2, 4, 6, 7, 8, 11, 16, 18, 22, 25, 62 und 75 von Escherichia coli verwendet werden.

6. Polyvalenter Impfstoff nach Anspruch 3, dadurch gekennzeichnet, dass er aus einer Mischung besteht, die mehrere Konjugate aus serotypspezifischen O-Polysacchariden von Pseudomonas aeruginosa und von Escherichia coli mit einem Exoprotein, bestehend aus Toxin A von Pseudomonas aeruginosa, einem Tetanustoxoid oder Diphtherietoxoid, enthält und dass er Antikörper gegen die entsprechenden Serotypen von Pseudomonas aeruginosa und Escherichia coli erzeugt.

7. Verwendung von Konjugatimpfstoffen gemäss Ansprüchen 1, 3, 4, 5 oder 6 zur Herstellung von Hyperimmunseren, welche ihrerseits zur Herstellung von intravenös oder intramuskulär verabreichbarem Immunglobulin dienen.

**Revendications**

1. Vaccin conjugué non toxique actif contre les infections par les bactéries de types *Pseudomonas aeruginosa* et *Escherichia coli,* constitué d'un O-polysaccharide dépourvu de lipide A, spécifique de l'espèce et du sérotype, provenant de l'endotoxine de *Pseudomonas aeruginosa* ou *Escherichia coli,* et d'une protéine, qui sont liés l'un à l'autre par une liaison covalente, caractérisé en ce que la protéine est une exotoxine ou exo-anatoxine bactérienne constituée de toxine A de *Pseudomonas aeruginosa,* d'une anatoxine tétanique ou diphtérique.

2. Procédé de préparation d'un vaccin conjugué selon la revendication 1, caractérisé en ce qu'on lie de façon covalente un O-polysaccharide dépourvu de lipide A, provenant de l'endotoxine de *Pseudomonas aeruginosa* ou *Escherichia coil,* avec une exoprotéine constituée de toxine A de *Pseudomonas aeruginosa* ou *Escherichia coli,* avec une exoprotéine constituée de toxine A de *Pseudomonas aeruginosa,* d'une anatoxine tétanique ou diphtérique.

3. Vaccin conjugué selon la revendication 1, caractérisé en ce qu'il se compose d'un mélange de 3 à 15 conjugués, formé de 3 à 15 O-polysaccharides dépourvues de lipide A, sérotypo-spéciques, provenant de l'endotoxine de différentes souches de *Pseudomonas aeruginosa* ou *Escherichia coli,* qui sont reliés isolément de façon covalente avec une exotoxine ou exo-anatoxine constituée de la toxine A de *Pseudomonas aeruginosa,* d'une anatoxine tétanique ou diphtérique.

4. Vaccin conjugué selon la revendication 3, caractérisé en ce que pour sa préparation on utilise des O-polysaccharides au moins des sérotypes O PA 220 (Habs Serotyp 6d), E 576 (Habs Serotyp 2ab), 6511 (Habs Serotyp 4), W 18 (Habs Serotyp 10), It 2 (Habs Serotyp 11), 8505 (Habs Serotyp 3, PA 53 (Habs Serotyp 1) et It 6 (Habs Serotyp 8) de *Pseudomonas aeruginosa.*

5. Vaccin conjugué selon la revendication 3, caractérisé en ce que pour sa préparation on utilise des O-polysaccharides au moins des sérotypes 1, 2, 4, 6, 7, 8, 11, 16, 22, 25, 62 et 75 d'*Escherichia coli.*

6. Vaccin polyvalent selon la revendication 3, caractérisé en ce qu'il se compose d'un mélange qui contient plusieurs conjugués de O-polysaccharides sérotypo-spécifiques de *Pseudomonas aeruginosa* et *Escherichia coli* avec une exoprotéine constituée de toxine A de *Pseudomonas aeruginosa,* d'une anatoxine tétanique ou diphtérique, et en ce qu'il produit des anticorps contre les sérotypes correspondants de *Pseudomonas aeruginosa* et *Escherichia coli.*

7. Application de vaccins conjugués selon les revendications 1, 3, 4, 5 ou 6 à la préparation de sera hyperimmuns, qui de leur côté servent à la préparation d'immunoglobuline administrable par voie intraveineuse ou intramusculaire.

**Claims**

1. Non-toxic conjugate vaccine effective against infections by Pseudomonas aeruginosa and Escherichia coli bacteria consisting of a species- and serotype-specific lipid-A-free O-polysaccharide from the endotoxin of Pseudomonas aeruginosa or Escherichia coli and of a protein, which are linked together covalently, characterized in that the protein is a bacterial exotoxin or exotoxoid consisting of toxin A of Pseudomonas aeruginosa, or of a tetanus or diphtheria toxoid.

2. Process for the preparation of a conjugate vaccine according to Claim 1, characterized in that a lipid-A-free O-polysaccharide from the endotoxin of Pseudomonas aeruginosa or Escherichia coli is covalently linked to an exoprotein consisting of toxin A of Pseudomonas aeruginosa, or of a tetanus or diphtheria toxoid.

3. Conjugate vaccine according to Claim 1, characterized in that it consists of a mixture of 3 to 15 conjugates formed from 3 to 15 serotype-specific lipid-A-free O-polysaccharides from the endotoxin of different strains of Pseudomonas aeruginosa or Escherichia coli which are individually covalently linked to an exotoxin or exotoxoid consisting of the toxin A of Pseudomonas aeruginosa, or of a tetanus toxoid or diphtheria toxoid.

4. Conjugate vaccine according to Claim 3, characterized in that O-polysaccharides from at least the O-serotypes PA 220 (Habs serotyp 6d), E 576 (Habs serotyp 2ab), 6511 (Habs serotyp 4), W 18 (Habs serotyp 10), It 2 (Habs serotyp 11), 8505 (Habs serotyp 3), PA 53 (Habs serotyp 1) and It 6 (Habs serotyp 8) of Pseudomonas aeruginosa are used for the preparation thereof.

5. Conjugate vaccine according to Claim 3, characterized in that O-polysaccharides from at least the O-serotypes 1, 2, 4, 6, 7, 8, 11, 16, 18, 22, 25, 62 and 75 of Escherichia coli are used for the preparation thereof.

6. Polyvalent vaccine according to Claim 3, characterized in that it consists of a mixture which contains several conjugates of serotype-specific O-polysaccharides of Pseudomonas aeruginosa and of Escherichia coli with an exoprotein consisting of toxin A of Pseudomonas aeruginosa, or of a tetanus toxoid or diphtheria toxoid, and that it raises antibodies against the corresponding serotypes of Pseudomonas aeruginosa and Escherichia coli.

7. Use of conjugate vaccines according to Claims 1, 3, 4, 5 or 6 for the preparation of hyperimmune sera which in turn are used for the preparation of immunoglobulin which can be administered intravenously or intramuscularly.